# EUROPEAN PATENT SPECIFICATION

(11) **EP 1 844 141 B1**
(45) Date of publication and mention of the grant of the patent: **11.07.2012**
(21) Application number: 06713360.3
(22) Date of filing: 02.02.2006
(51) Int. Cl.: C12N 15/10

(54) **METHOD FOR SEPARATING AND PURIFYING NUCLEIC ACID**
VERFAHREN ZUR TRENNUNG UND REINIGUNG VON NUKLEINSÄURE
PROCÉDÉ SERVANT À ISOLER ET À PURIFIER UN ACIDE NUCLÉIQUE

(30) Priority: 04.02.2005 JP 2005028991
(43) Date of publication of application: 17.10.2007
(73) Proprietor: Kurashiki Boseki Kabushiki Kaisha, Kurashiki-shi Okayama (JP)
(72) Inventor: SASAKI, Tasuku, c/o FUJIFILM Corporation, Asaka-shi, Saitama (JP); INOMATA, Hiroko, c/o FUJIFILM Corporation, Asaka-shi, Saitama (JP); IWATA, Rie, c/o FUJIFILM Corporation, Asaka-shi, Saitama (JP)
(74) Representative: Hiebl, Inge Elisabeth
(86) International application number: PCT/JP2006/302217
(87) International publication number: WO 2006/083017

(56) References cited:
- JP-A- 09 327 291
- JP-A- 2001 136 970
- JP-A- 2003 128 691
- JP-A- 2004 290 150
- US-A1- 2003 170 664

## Description

### Technical Field

The present invention relates to a method of separating and purifying nucleic acid, and more particularly to a method of separating and purifying nucleic acid from a mixture containing nucleic acid.

### Background Art

Nucleic acid is utilized in various forms in various fields. For example in the field of recombinant nucleic acid, it is required to use nucleic acid in the form of a probe, genome nucleic acid or plasmid nucleic acid.

Also in diagnostic field, nucleic acid is utilized in various forms for various purposes. For example a nucleic acid probe is commonly utilized for detection and diagnosis of human pathogens. Similarly nucleic acid is used for detecting a genetic lesion, and also for detecting a food contaminant. Furthermore, nucleic acid is widely utilized in positional confirmation, identification and isolation of a desired nucleic acid for various purposes ranging from a genetic mapping to cloning or recombinant expression.

In most cases, the nucleic acid is available only in an extremely small amount, and requires complex and time-consuming operations for separation and purification. Such complex and time-consuming operations often lead to a loss in the nucleic acid. Also a purification of nucleic acid from a sample obtained from serum, urine or a bacteria culture involves possibilities of resulting in a contamination or a false positive result.

A widely known separation-purification method is based on adsorbing nucleic acid on a solid phase such as silicon dioxide, a silica polymer or magnesium silicate, followed by operations of washing, desorption and the like (for example cf. JP-B-7-51065). This method provides a satisfactory separating ability but is insufficient in the simplicity, rapidity and adaptability to an automatic operation. Also equipment and apparatus employed in this method are unsuitable for an automation and a size reduction, and particularly an adsorbent member involves drawbacks of being difficult to mass produce industrially with a constant performance, inconvenient in handling and difficult to produce in various forms. Also since it requires a certain thickness for obtaining a mechanical strength because of the brittleness of the material itself, it is necessary, in selectively recovering RNA from a mixed sample of DNA and RNA, to utilize an expensive reagent such as DNase.

Also a known simple and efficient separation-purification of nucleic acid employs a solution for adsorbing nucleic acid on a solid phase and a solution for desorbing nucleic acid from the solid phase, and conducts separation-purification of nucleic acid by adsorbing nucleic acid on a solid phase formed by an organic polymer having a hydroxyl group on a surface and desorbing nucleic acid therefrom (JP-A-2003-128691 and JP-A-2004-49108).

Other known methods for separation-purification of nucleic acid include a centrifuging method, a method utilizing magnetic beads and a filteration method, and apparatuses for separation-purification of nucleic acid are proposed based on these methods. For example, as a nucleic acid-separating apparatus based on the filteration method, there is proposed a mechanism in which a plurality of filter tubes, each accommodating a filter, are set on a rack, then a sample solution containing nucleic acid is separately injected therein, then a periphery of the bottom part of the rack is tightly closed, with a sealant, in an air chamber, of which the interior is then reduced in pressure to simultaneously suck all the filter tubes from exit sides thereof to pass the sample solution through the filters and to adsorb nucleic acid on the filters, and a washing solution and a recovering solution are separately injected and similarly sucked under a reduced pressure to achieve washing and desorption of the nucleic acid (for example cf. Japanese Patent No. 2832586).

In case of adsorbing nucleic acid on a porous membrane formed for example of an organic polymer having a hydroxyl group on the surface, a solution containing a water-soluble organic solvent is usually added to a solution containing nucleic acid. The water-soluble organic solvent employed in such addition is usually an aqueous solution of ethanol. In a solution used for adsorbing nucleic acid on the solid phase, after such addition to the solution containing nucleic acid, a final ethanol concentration (end ethanol concentration) is generally preferred within a range of 20 to 60 mass%.

US 2003/0170664 discloses a method for isolating and purifying an nucleic acid comprising the step of adsorbing a nucleic acid onto a solid phase composed of an organic high polymer having a hydroxide group on a surface thereof, and desorbing the nucleic acid from the solid phase.

### Disclosure of the Invention

An object of the present invention is to provide, in the method of separating and purifying nucleic acid by adsorbing nucleic acid in a biomaterial on a surface of a solid phase and, after washing and the like, desorbing nucleic acid, a method capable of processing a larger amount of biomaterial without prolonging a time for obtaining a solution for nucleic acid adsorption on the solid phase.

The above-mentioned object can be attained by following constitutions of the present invention:
1. A method for separating and purifying a nucleic acid comprising steps of:
   (1) adding a lysis solution to a biomaterial to prepare a sample solution containing a nucleic acid, and
      adding a water-soluble organic solvent or a solution containing a water-soluble organic solvent to the sample solution thereby preparing a sample solution containing the water-soluble organic solvent;
   (2) contacting the sample solution containing the water-soluble organic solvent with a solid phase thereby adsorbing the nucleic acid on the solid phase;
   (3) contacting a washing solution with the solid phase thereby washing the solid phase in a state where the nucleic acid is adsorbed on the solid phase; and
   (4) contacting a recovering solution with the solid phase thereby desorbing the nucleic acid from the solid phase,
   wherein, in the step (1), the water-soluble organic solvent or the solution containing the water-soluble organic solvent is added separately in at least two batches,
   wherein the amount of first addition is 5 to 90 vol.% of a total addition amount, and
   wherein the step (1) further comprises:
   after adding the water-soluble organic solvent or the solution containing the water-soluble organic solvent at least once to the sample solution, agitating the sample solution by an operation including at least one of a shaking, an inverting and a rotating movement; and
      further adding the water-soluble organic solvent or the solution containing the water-soluble organic solvent at least once to the sample solution after the agitation.
2. The method for separating and purifying a nucleic acid according to item 1,
   wherein the step (1) comprises:
   after adding the water-soluble organic solvent or the solution containing the water-soluble organic solvent at least once to the sample solution, agitating the sample solution by an operation including at least a suction and a discharge of the solution, and
   further adding the water-soluble organic solvent or the solution containing the water-soluble organic solvent at least once to the sample solution after the agitation,
   whereby in case of conducting both the operation including at least one of a shaking, an inverting and a rotating movement and the operation including at least a suction and a discharge of the solution, these operations may be conducted in an arbitrary order.
3. The method for separating and purifying a nucleic acid according to any of items 1 or 2,
   wherein, in the step (1), the sample solution containing the water-soluble organic solvent has a concentration of the water-soluble organic solvent within a range of from 5 to 90 mass%.
4. The method for separating and purifying a nucleic acid according to any of items 1 or 2,
   wherein, in the step (1), the sample solution containing the water-soluble organic solvent has a concentration of the water-soluble organic solvent within a range of from 10 to 60 mass%.
5. The method for separating and purifying a nucleic acid according to any of items 1 or 2,
   wherein, in the step (1), the sample solution containing the water-soluble organic solvent has a concentration of the water-soluble organic solvent within a range of from 20 to 40 mass%.
6. The method for separating and purifying a nucleic acid according to any of items 1 to 5,
   wherein the solid phase is a porous membrane comprising an organic polymer that adsorbs a nucleic acid by an interaction substantially not involving an ionic bonding.
7. The method for separating and purifying a nucleic acid according to item 6,
   wherein the organic polymer has a hydroxyl group.
8. The method for separating and purifying a nucleic acid according to item 6 or 7,
   wherein the porous membrane comprises an organic material obtained by saponification of a mixture of acetylcelluloses different in acetyl value.
9. The method for separating and purifying a nucleic acid according to any of items 6 to 8,
   wherein the porous membrane has a front surface and a back surface asymmetrical with each other.
10. The method for separating and purifying a nucleic acid according to any of items 1 to 9,
   wherein the lysis solution is a nucleic acid-solubilizing reagent.
11. The method for separating and purifying a nucleic acid according to any of items 1 to 10,
   wherein the biomaterial is an animal tissue.
12. The method for separating and purifying a nucleic acid according to item 10,
   wherein the nucleic acid-solubilizing reagent comprises at least one selected from the group consisting
   of a chaotropic salt, a nucleic acid-stabilizing agent, a surfactant, a buffer and a defoaming agent.
13. The method for separating and purifying a nucleic acid according to item 12,
   wherein the chaotropic salt comprises at least one selected from the group consisting of guanidine hydrochloride and guanidine thiocyanate.
14. The method for separating and purifying a nucleic acid according to any of items 1 to 13,
   wherein the water-soluble organic solvent is at least one selected from the group consisting of methanol, ethanol, propanol or an isomer thereof and butanol or an isomer thereof.
15. The method for separating and purifying a nucleic acid according to any of items 1 to 14,
   wherein the washing solution comprises at least one selected from the group consisting of methanol, ethanol, propanol or an isomer thereof and butanol or an isomer thereof, in an amount of from 20 to 50 mass.
16. The method for separating and purifying a nucleic acid according to any of items 1 to 15,
   wherein the washing solution is a solution comprising a chloride salt in an amount of from 10 mmol/L to 1 mol/L.
17. The method for separating and purifying a nucleic acid according to any of items 8 to 16,
   wherein, in the steps (2), (3) and (4), passing of the sample solution containing the water-soluble organic solvent, the washing solution or the recovering solution through the porous membrane is conducted by utilizing: a nucleic acid separating-purifying cartridge that receives the porous membrane which a solution can pass through in an inside of a container having at least two openings; and a pressure generating apparatus that is a pump detachably mountable on one of the at least two openings of the nucleic acid separating-purifying cartridge.

In the present invention, in a step of preparing a solution for adsorbing nucleic acid on a solid phase ("sample solution containing water-soluble organic solvent" in the invention), the water-soluble organic solvent is reduced in an amount of addition and is not changed in an end concentration, thus being employed in a concentration higher than in the ordinary case. Therefore the solution for adsorbing nucleic acid on the solid phase can be reduced in the final amount, so that the lysis solution initially added to the biomaterial can be increased. In the present invention, the water-soluble organic solvent is separately added in plural portions (batches), and a mixing can be facilitated even with the water-soluble organic solvent of a higher concentration. It is thus rendered possible to process a larger amount of the biomaterial and to promptly recover nucleic acid. The solid phase to be employed is not particularly restricted but is preferably constituted of a porous membrane, and it is preferable, for attaining the effect of the present invention, to employ a nucleic acid separation-purification cartridge accommodating such porous membrane and to employ, as such porous membrane, a membrane capable of adsorbing nucleic acid by an interaction not involving an ionic bonding, and more preferable to employ a nucleic acid separation-purification cartridge accommodating (receiving) a porous membrane of an organic polymer in a container having two openings.

### Best Mode For Carrying Out the method:

The method for separating and purifying nucleic acid at least includes:
(1) a step of adding a lysis solution to a biomaterial to prepare a sample solution, and
   adding a water-soluble organic solvent or a solution containing a water-soluble organic solvent to the sample solution thereby preparing a sample solution containing the water-soluble organic solvent (hereinafter also called "step of preparing a sample solution containing water-soluble organic solvent");
(2) a step of contacting the sample solution containing the water-soluble organic solvent with a solid phase thereby causing nucleic acid to be adsorbed on the solid phase (hereinafter also called "adsorbing step");
(3) a step of contacting a washing solution with the solid phase thereby washing the solid phase in a state where the nucleic acid is adsorbed thereon (hereinafter also called "washing step"); and
(4) a step of contacting a recovering solution with the solid phase thereby desorbing the nucleic acid from the solid phase (hereinafter also called "recovering step").

The solid phase to be used is not particularly restricted, but is preferably a porous membrane which adsorbs nucleic acid by an interaction substantially not involving an ionic bonding (hereinafter also called "nucleic acid-adsorbing porous membrane").

In the step (2), (3) or (4), the sample solution containing the water-soluble organic solvent, the washing solution or the recovering solution is preferably passed through the nucleic acid-adsorbing porous membrane by a pressure generating apparatus, and, more preferably the step (2), (3) or (4) is conducted by injecting the sample solution containing the water-soluble organic solvent, the washing solution or the recovering solution into one of the openings of a nucleic acid separation-purification cartridge having at least two openings and accommodating therein the nucleic acid-adsorbing porous membrane, and pressurizing the interior of the cartridge by a pressure generating apparatus coupled with the above-mentioned one opening thereby causing the injected liquid to pass through the membrane and to be discharged from the other opening. By passing the sample solution containing the water-soluble organic solvent, the washing solution or the recovering solution through the porous membrane under a pressurized state, the apparatus can be advantageously automated in compact manner. The pressurization with the pump is conducted within a range of 10 to 300 kPa, more preferably 40 to 200 kPa.

More preferably, nucleic acid is separated and purified with the nucleic acid separation-purification cartridge accommodating the nucleic acid-adsorbing porous membrane, by following steps:
(a) a step of injecting a sample solution containing a water-soluble organic solvent into one of the openings of a nucleic acid separation-purification cartridge having at least two openings and accommodating therein a nucleic acid-adsorbing porous membrane through which a solution can pass;
(b) a step of pressurizing the interior of the nucleic acid separation-purification cartridge by a pressure generating apparatus coupled with the above-mentioned one opening thereby causing the injected sample solution containing the water-soluble organic solvent to pass through the membrane and to be discharged from the other opening of the nucleic acid separation-purification cartridge, thereby causing nucleic acid to be adsorbed in the nucleic acid-adsorbing porous membrane;
(c) a step of injecting a washing solution into the one opening of the nucleic acid separation-purification cartridge;
(d) a step of pressurizing the interior of the nucleic acid separation-purification cartridge by the pressure generating apparatus coupled with the one opening thereby causing the injected washing solution to pass through the membrane and to be discharged from the other opening of the nucleic acid separation-purification cartridge, thereby washing the nucleic acid-adsorbing porous membrane in a state where nucleic acid is adsorbed therein;
(e) a step of injecting a recovering solution into the one opening of the nucleic acid separation-purification cartridge; and
(f) a step of pressurizing the interior of the nucleic acid separation-purification cartridge by the pressure generating apparatus coupled with the one opening thereby causing the injected recovering solution to pass through the membrane and to be discharged from the other opening of the nucleic acid separation-purification cartridge, thereby desorbing nucleic acid from the nucleic acid-adsorbing porous membrane and discharging it from the nucleic acid separation-purification cartridge.

This process for separating and purifying nucleic acid, from the initial step of injecting the sample solution containing the water-soluble organic solvent to the step of obtaining nucleic acid outside the nucleic acid separation-purification cartridge, can be completed substantially within 30 minutes, and within 2 minutes in a preferred situation.

Also this process for separating and purifying nucleic acid allows to recover nucleic acid with a purity, in a measured value (260 nm/280nm) by an ultraviolet-visible spectrophotometer, of 1.6 to 2.0 in case of DNA and 1.8 to 2.2 in case of RNA, thus constantly providing nucleic acid of a high purity with a low contamination by impurities. It is further possible to recover nucleic acid with a purity, in a measured value (260 nm/280nm) by an ultraviolet-visible spectrophotometer, of about 1.8 in case of DNA and about 2.0 in case of RNA.

Also in the above-described process, the pressure generating apparatus can be a syringe, a pipetter or a pump capable of pressurization such as a perista pump, or an apparatus capable of pressure reduction such as an evaporator. Among these, a syringe is suitable for a manual operation, and a pump is suitable for an automatic operation. Also a pipetter has an advantage of easily allowing a single-hand operation. The pressure generating apparatus is preferably detachably coupled with the one opening of the nucleic acid separation-purification cartridge.

The above-described process can also be advantageously conducted by reducing the pressure of the interior of the nucleic acid separation-purification cartridge by a pressure generating apparatus coupled with the other opening of the nucleic acid separation-purification cartridge. It can also be advantageously conducted by applying a centrifugal force to the nucleic acid separation-purification cartridge.

The biomaterial to be employed in the present invention is not particularly restricted as long as it contains nucleic acid, and includes cells, a tissue, blood and bacteria. For example in the diagnostic field, it can be a body liquid obtained as a biomaterial such as whole blood, blood plasma, serum, urine, faece, semen or saliva, a plant (or a part thereof), an animal (or a part thereof), bacteria, viruses or cultured cells, or a liquid such as a solution or a homogenate prepared from such biomaterial. The cultured cells include floating cells and adherent cells. The floating cells mean those grow and proliferate in a floating state in a culture medium without adhering to a wall of a culture cell, and representative strains include HL60, U937 and HeLaS3. The adherent cells mean those grow and proliferate in a state adhering to a culture cell wall in a culture medium, and representative strains include NIH3T3, HEK293, HeLa, COS and CHO. An animal (or a part thereof) to be employed as the biomaterial can be an animal tissue, and any tissue constituting an individual animal and collectible by anatomy or biopsy of the animal, such as a liver, a kidney, a spleen, a brain, a heart, a lung or a thymus. Hereinafter such biomaterial may also be called an analyte.
(1) A step of adding a lysis solution to a biomaterial to prepare a sample solution, and adding a water-soluble organic solvent or a solution containing a water-soluble organic solvent to the sample solution thereby preparing a sample solution containing the water-soluble organic solvent ("step of preparing a sample solution containing water-soluble organic solvent") :

At first a lysis solution is added to an analyte to obtain "sample solution containing nucleic acid". The lysis solution is preferably an aqueous solution containing a reagent capable of dissolving nucleic acid (nucleic acid-solubilizing reagent) to conduct a process of dissolving a cell membrane and a nuclear envelope. Then a water-soluble organic solvent or a solution containing a water-soluble organic solvent is added to disperse nucleic acid in the aqueous solution, thereby obtaining "sample solution containing nucleic acid".

The nucleic acid-solubilizing reagent can be a solution containing at least one of a chaotropic salt, a nucleic acid-stabilizing agent, a surfactant, a buffer and a defoaming agent.

The chaotropic salt in the nucleic acid-solubilizing reagent preferably has a concentration of 0.5 mol/L or higher, more preferably 0.5 to 8 mol/L and further preferably 1 to 6 mol/L. Any known chaotropic salt may be employed without particular restriction. The chaotropic salt can be a guanidine salt, sodium isocyanate, sodium iodide or potassium iodide, among which a guanidine salt is preferred. The guanidine salt can be guanidine hydrochloride, guanidine isocyanate, or guanidine thiocyanate, among which guanide hydrochloride is preferable. Such salt may be employed singly or in a combination of plural kinds.

The surfactant in the nucleic acid-solubilizing reagent can be, for example, a nonionic surfactant, a cationic surfactant, an anionic surfactant or an amphoteric surfactant. In the invention, a nonionic surfactant is preferably employed. The nonionic surfactant can a surfactant of polyoxyethylene alkylphenyl ether type, a surfactant of polyoxyethylene alkyl ether type, or a fatty acid alkanolamide, preferably a surfactant of polyoxyethylene alkyl ether type. More preferably the surfactant of polyoxyethylene alkyl ether type is selected from POE decyl ether, POE lauryl ether, POE tridecyl ether, POE alkylenedecyl ether, POE sorbitan monolaurate, POE sorbitan monooleate, POE sorbitan monostearate, polyoxyethylene sorbit tetraoleate, POE alkylamine, and POE acetylene glycol.

Also a cationic surfactant can be employed preferably. More preferably the cationic surfactant is selected from cetyltrimethyl ammonium bromide, dodecyltrimethyl ammonium chloride, tetradecyltrimethyl ammonium chloride and cetylpyridinium chloride. Such surfactant may be employed singly or in a combination of plural kinds. In the solution of the nucleic acid-solubilizing reagent, such surfactant preferably has a concentration of 0.1 to 20 mass%. (In this specification, mass% is equal to weight%.)

The nucleic acid-solubilizing reagent is preferably used in combination with a nucleic acid-stabilizing agent. The analyte may contain nuclease or the like which decomposes nucleic acid and which, upon homogenization of the analyte, acts on nucleic acid thereby reducing the yield thereof. In order to avoid such phenomenon, a stabilizing agent capable of deactiving nuclease may be added to the nucleic acid-solubilizing solution.

In this manner it is rendered possible to improve a recovered amount and a recovery efficiency of nucleic acid, thereby reducing the amount of the analyte and enabling a faster process.

As the deactivating agent for nuclease, generally a reducing agent is employed advantageously. The reducing agent can be a hydride such as hydrogen, hydrogen iodide, hydrogen sulfide, aluminum lithium hydride, or boron sodium hydride; an electrically strongly positive metal such as an alkali metal, magnesium, calcium, aluminum, zinc, or an amalgam thereof, or an organic oxide such as an aldehyde, a saccharide, formic acid or oxalic acid, but a mercapto compound is preferred. The mercapto compound can be N-acetyl cysteine, mercaptoethanol or an alkylmercaptane but is not particularly restricted. The mercapto compound may be employed, in the lysis solution, with a concentration of 0.1 to 20 mass%, preferably 0.5 to 15 mass%.

The buffer can be an ordinary pH buffer, preferably a pH buffer for biochemical use. Such buffer includes a buffer containing a citrate salt, a phosphate salt or an acetate salt, tris-HCl, TE (tris-HCl/EDTA), TBE (tris-borate/EDTA), TAE (tris-acetate/EDTA), or a Good's buffer. The Good's buffer can be, for example, MES (2-morpholinoethanesulfonic acid), Bis-Tris (bis(2-hydroxyethyl)iminotris(hydroxymethyl)methane), HEPES (2-[4-(2-hydroxyethyl)-1-piperazinyl]ethanesulfonic acid), PIPES (piperazine-1,4-bis(2-ethanesulfonic acid)), ACES (N-(2-acetamino)-2-aminoethanesulfonic acid), CAPS (N-cyclohexyl-3-aminopropanesulfonic acid), or TES (N-tris(hydroxymethyl)methyl-2-aminoethanesulfonic acid).

Such buffer is employed in the nucleic acid-solubilizing reagent with a concentration preferably of 1 to 300 mmol/L.

The nucleic acid-solubilizing reagent may preferably contain a defoaming agent. The defoaming agent is preferably a silicone-type defoaming agent or an alcohol-type defoaming agent, and the alcohol-type defoaming agent is preferably an acetylene glycol surfactant.

Specific examples of the defoaming agent include a silicone-type defoaming agent (such as silicone oil, dimethylpolysiloxane, silicone emulsion, denatured polysiloxane, or silicone compound), an alcohol-type defoaming agent (such as acetylene glycol, heptanol, ethylhexanol, a higher alcohol or polyoxyalkylene glycol), an ether-type defoaming agent (such as heptyl cellosolve, or nonyl cellosolve-3-heptylsorbitol), an oil/fat-type defoaming agent (such as animal oil or vegetable oil), a fatty acid-type defoaming agent (such as stearic acid, oleic acid, or palmitic acid), a metal soap-type defoaming agent (such as aluminum palmitate, or calcium stearate), a fatty acid ester-type defoaming agent (such as a natural wax, or tributyl phosphate), a phosphate ester-type defoaming agent (such as sodium octylphosphate), an amine-type defoaming agent (such as diamylamine), an amide-type defoaming agent (such as stearylamide), and other defoaming agents (such as ferric sulfate or bauxite). Particularly preferably, a silicone-type defoaming agent and an alcohol-type defoaming agent may be used in combination as the defoaming agent. Also an acetylene glycol-type surfactant may be preferably employed as the alcohol-type defoaming agent.

Also the solution of the nucleic acid-solubilizing reagent may contain a water-soluble organic solvent. Such water-soluble organic solvent is used for increasing solubility of various reagents contained in the nucleic acid-solubilizing reagent, and can be acetone, chloroform or dimethylformamide, but is preferably an alcohol. Such alcohol can be a primary, secondary or tertiary alcohol.

More preferably, the alcohol can be methanol, ethanol, propanol or an isomer thereof, or a butanol or an isomer thereof. Such water-soluble organic solvent may be employed singly or in a combination of plural kinds. In the solution of the nucleic acid-solubilizing reagent, the water-soluble organic solvent preferably has a concentration of 1 to 20 mass%.

The solution of the nucleic acid-solubilizing reagent is preferably has a pH value of 3 to 8, more preferably 4 to 7 and further preferably 5 to 7.

The analyte is preferably subjected to a homogenization process, thereby improving an adaptability to an automated process. The homogenization can be achieved for example by an ultrasonic treatment, a process of utilizing sharp projections, a high-speed agitation or extrusion through a fine gap, or a process utilizing beads of glass, stainless steel or zirconia.

A method for mixing the homogenized analyte and the nucleic acid-solubilizing reagent nucleic acid is not particularly restricted. The mixing is preferably conducted by an agitating apparatus of 30 to 3,000 rpm, for a period of 1 second to 3 minutes. It is thus possible to increase an yield of the separated and purified nucleic acid. The mixing is also preferably achieved by conducting an inverting 5 to 30 times. The mixing can also be achieved by conducting a pipetting operation 10 to 50 times. In this case, the yield of the separated and purified nucleic acid can be increased by a simple operation.

The obtained sample solution containing nucleic acid is subjected to an addition of a water-soluble organic solvent or an aqueous solution of a water-soluble organic solvent, thereby obtaining a sample solution containing a water-soluble organic solvent. In the invention, the water-soluble organic solvent or the solution containing the water-soluble organic solvent is added in separate manner in at least two portions. The water-soluble organic solvent to be added to the sample solution containing nucleic acid can preferably be an alcohol, which can be a primary, secondary or tertiary alcohol and which is preferably methanol, ethanol, propanol, butanol or an isomer thereof. In the sample solution containing the water-soluble organic solvent, the water-soluble organic solvent preferably has a final concentration of 5 to 90 mass%, more preferably 10 to 60 mass% and particularly preferably 20 to 40 mass%. The water-soluble organic solvent or the aqueous solution of the water-soluble organic solvent to be added has a concentration of 20 to 100 vol.%, preferably 50 to 100 vol.%. In the invention, the water-soluble organic solvent or the solution containing the water-soluble organic solvent is added in separate manner in at least two portions. An amount of first addition is 5 to 90 vol.% of a total addition amount, preferably 25 to 70 vol.%.

The above step (1) comprises after adding the water-soluble organic solvent or the solution containing the water-soluble organic solvent at least once to the sample solution, to conduct an agitation by an operation including at least one of a shaking, an inverting and a rotating movement, and to further add, to the solution after agitation, a water-soluble organic solvent or a solution containing a water-soluble organic solvent at least once. It is preferable, after adding the water-soluble organic solvent or the solution containing the water-soluble organic solvent at least once to the sample solution, to conduct an agitation by an operation including at least a suction and a discharge of the solution, and to further add, to the solution after agitation, a water-soluble organic solvent or a solution containing a water-soluble organic solvent at least once. In case of conducting both the operation including at least one of a shaking, an inverting and a rotating movement and the operation including at least a suction and a discharge of the solution, these operations may be conducted in an arbitrary order. The suction and discharge of the solution can be advantageously conducted for example with a pipette.

Also such agitation may be conducted after a second addition of the water-soluble organic solvent or the solution containing the water-soluble organic solvent.

The obtained sample solution containing the water-soluble organic solvent preferably has a surface tension of 0.05 J/cm² or less, a viscosity of 1 to 10,000 mPa, and a specific gravity of 0.8 to 1.2. Such physical properties facilitates removal of the sample solution containing the water-soluble organic solvent after a contact thereof with a nucleic acid-adsorbing porous membrane.
(2) A step of contacting the sample solution containing the water-soluble organic solvent with a solid phase thereby causing nucleic acid to be adsorbed on the solid phase ("adsorbing step"):

In the following a solid phase to be employed in the invention and the adsorbing step thereof will be explained.

A solid phase to be employed in the invention is preferably capable of adsorbing nucleic acid by an interaction substantially not involving an ionic bonding. This means that the solid phase is not "ionized" in a condition of use thereof, and the nucleic acid and the solid phase are assumed to attract each other by a change in the polarity of the environment. It is thus possible to separate and purity nucleic acid with an excellent separating ability and a satisfactory washing efficiency. The solid phase preferably has a hydrophilic group, whereby the nucleic acid and the solid phase are assumed to attract each other by a change in the polarity of the environment.

The hydrophilic group indicates a polar group (atomic group) capable of an interaction with water, and includes all the groups (atomic groups) involved in nucleic acid adsorption. The hydrophilic group is preferably a group having a medium interaction with water (cf. "group with a medium hydrophilicity" in "hydrophilic group", *Kagaku Dai-jiten* (Encyclopaedia Chimica), published by Kyoritsu Shuppan Co.), and can be, for example, a hydroxyl group, a carboxyl group, a cyano group, or an oxyethylene group, and preferably a hydroxyl group.

A solid phase having a hydrophilic group means a solid phase in which a material itself constituting the solid phase has a hydrophilic group, or a solid phase in which a hydrophilic group is introduced into a material constituting the solid phase by a treatment or a coating. The material constituting the solid phase may be an organic material or an inorganic material. For example there may be employed a solid phase of which a constituent material is an organic material having a hydrophilic group, a solid phase in which a hydrophilic group is introduced by treating a solid phase of an organic material without a hydrophilic group, a solid phase in which a hydrophilic group is introduced by coating a solid phase of an organic material without a hydrophilic group, with a material having a hydrophilic group, a solid phase of which a constituent material is an inorganic material having a hydrophilic group, a solid phase in which a hydrophilic group is introduced by treating a solid phase of an inorganic material without a hydrophilic group, or a solid phase in which a hydrophilic group is introduced by coating a solid phase of an inorganic material without a hydrophilic group, with a material having a hydrophilic group.

A solid phase of a material having a hydroxyl group can be a solid phase formed by polyhydroxyethylacrylic acid, polyhydroxyethylmethacrylic acid, polyvinylalcohol, polyvinylpyrrolidone, polyacrylic acid, polymethacrylic acid, polyoxyethylene, acetylcellulose or a mixture of acetylcelluloses with difference acetyl values, and preferably a solid phase of an organic material having a hydroxyl group.

The solid phase of an organic material having a hydroxyl group is preferably of a material having a polysaccharide structure, and more preferably a solid phase of an organic polymer formed by a mixture of acetylcelluloses different in acetyl value. The mixture of acetylcelluloses different in acetyl value is preferably a mixture of triacetylcellulose and diacetylcellulose, a mixture of triacetylcellulose and monoacetylcellulose, a mixture of triacetylcellulose, diacetylcellulose and monoacetylcellulose, or a mixture of diacetylcellulose and monoacetylcellulose, particularly preferably a mixture of triacetylcellulose and diacetylcellulose. A mixing ratio of triacetylcellulose and diacetylcellulose is preferably 99:1 to 1:99, more preferably 90:10 to 50:50.

A more preferable organic material having a hydroxyl group is a saponified substance of acetylcellulose described in JP-A-2003-128691. A saponified substance of acetylcellulose is obtained by a saponification of a mixture of acetylcelluloses different in acetyl value, and is preferably a saponified substance of a mixture of triacetylcellulose and diacetylcellulose, a saponified substance of a mixture of triacetylcellulose and monoacetylcellulose, a saponified substance of a mixture of triacetylcellulose, diacetylcellulose and monoacetylcellulose, or a saponified substance of a mixture of diacetylcellulose and monoacetylcellulose, more preferably a saponified substance of a mixture of triacetylcellulose and diacetylcellulose. A mixing ratio (mass ratio) of triacetylcellulose and diacetylcellulose is preferably 99:1 to 1:99, more preferably 90:10 to 50:50. In this case, an amount (density) of hydroxyl groups on the solid phase surface can be controlled by a level of saponification process (saponification rate). A higher amount (density) of the hydroxyl groups is preferable for increasing the separating efficiency of nucleic acid. For example, in case of an acetylcellulose such as triacetylcellulose, the saponification rate (surface saponification rate) is preferably about 5 % or higher, and more preferably 10 % or higher. Also for increasing the surface area of the organic polymer having a hydroxyl group, it is preferable to saponify a solid phase of acetylcellulose.

A saponification indicates contacting acetylcellulose with a saponifying solution (for example an aqueous solution of sodium hydroxide). Thus, in an ester derivative of cellulose contacted with the saponifying solution, an ester group is hydrolyzed and a hydroxyl group is introduced to regenerate cellulose. The regenerated cellulose thus prepared is different from the original cellulose in a crystalline state and the like. Also the saponification rate can be varied with a saponification process under changes in the concentration of sodium hydroxide and in the process time. The saponification rate can be easily measured for example by NMR, IR or XPS (for example by a decrease of a peak of the carbonyl group).

For introducing a hydrophilic group into the solid phase of an organic material without a hydrophilic group, a graft polymer chain having a hydrophilic group in a polymer chain or in a side chain may be bonded to the solid phase. For bonding a graft polymer chain to the solid phase of the organic material, two methods are available, namely a method of chemically bonding the solid phase with a graft polymer chain and a method of polymerizing a compound having a polymerizable double bond starting from the solid phase thereby forming a graft polymer chain.

In the method of chemically bonding the solid phase with a graft polymer chain, the grafting can be conducted by utilizing a polymer having a functional group, capable of reacting with the solid phase, in a terminal end of the polymer or in a side chain, and causing a chemical reaction of the functional group with a functional group of the solid phase. The functional group capable of reacting with the solid phase is not particularly restricted as long as it is capable of reacting with the functional group of the solid phase, and can be, for example, a silane coupling group such as an alkoxysilane, an isocyanate group, an amino group, a hydroxyl group, a carboxyl group, a sulfonic acid group, a phosphoric acid group, an epoxy group, an allyl group, a methacryloyl group, or an acryloyl group. As the polymer having a reactive functional group in a terminal end of the polymer or in a side chain, particularly useful is a polymer having a trialkoxysilyl group in a terminal end of the polymer, a polymer having an amino group in a terminal end of the polymer, a polymer having a carboxyl group in a terminal end of the polymer, a polymer having an epoxy group in a terminal end of the polymer, or a polymer having an isocyanate group in a terminal end of the polymer. The polymer to be employed in this case can be any polymer having a hydrophilic group involved in the adsorption of nucleic acid, but can be, for example, polyhydroxyethylacrylic acid, polyhydroxyethylmethacrylic acid or a salt thereof; polyvinyl alcohol, polyvinylpyrrolidone, polyacrylic acid, polymethacrylic acid or a salt thereof; or polyoxyethylene.

The method of polymerizing a compound having a polymerizable double bond starting from the solid phase thereby forming a graft polymer chain is generally called a surface graft polymerization. The surface graft polymerization means a method of providing a surface of a base material with active species by a plasma irradiation, a light irradiation or a heating, and bonding a compound, having a polymerizable double bond and positioned so as to be contactable with the solid phase, with the solid phase by a polymerization. A compound useful for forming a graft polymer chain bonded to the base material is required to meet two characteristics of having a polymerizable double bond and having a hydrophilic group involved in the adsorption of nucleic acid. Such compound can be, as long as having a double bond within the molecule, a polymer, an oligomer, or a monomer having a hydrophilic group. A particularly useful compound is a monomer having a hydrophilic group. Specific examples of the particularly useful monomer having hydrophilic group include following monomers having a hydroxyl group, such as 2-hydroxyethyl acrylate, 2-hydroxyethyl methacrylate, and glycerol monomethacrylate. Also a carboxyl group-containing monomer such as acrylic acid or methacrylic acid, or an alkali metal salt or an amine salt thereof, can be employed advantageously.

As another method for introducing a hydrophilic group into a solid phase of an organic material not having a hydrophilic group, a material having a hydrophilic group may be coated. A material to be used for coating is not particularly restricted as long as it has a hydrophilic group involved in the adsorption of nucleic acid, but is preferably a polymer of an organic material, in consideration of ease of operation. Such polymer can be, for example, polyhydroxyethylacrylic acid, polyhydroxyethylmethacrylic acid or a salt thereof; polyvinyl alcohol, polyvinylpyrrolidone, polyacrylic acid, polymethacrylic acid or a salt thereof; polyoxyethylene, acetylcellulose or a mixture of acetylcelluloses different in acetyl value, and is preferably a polymer having a polysaccharide structure.

It is also possible, after coating the solid phase of an organic material not having a hydrophilic group with acetylcellulose or a mixture of acetylcelluloses different in acetyl value, to conduct a saponification process on the acetylcellulose or the mixture of acetylcelluloses different in acetyl value thus coated. In such case, the saponification rate is preferably about 5 % or higher, more preferably about 10 % or higher.

The solid phase of an inorganic material having a hydroxyl group can be, for example, a solid phase formed by a silica compound or the like. In case of use in a membrane form, it can be a glass filter. It may also be a porous silica film as described in Japanese Patent No. 3058342. Such porous silica film can be prepared by developing, on a base plate, a developing solution of a cationic amphiphilic substance having a bimolecular film-forming ability, then removing a solvent from the liquid film on the base plate thereby preparing a multi-layered film of bimolecular films of the amphiphilic substance, then contacting the multi-layered film of bimolecular films with a solution containing a silica compound and extracting the multi-layered film of bimolecular films.

For introducing a hydrophilic group into the solid phase of an inorganic material without a hydrophilic group, two methods are available, namely a method of chemically bonding the solid phase with a graft polymer chain and a method of polymerizing a monomer, having a hydrophilic group and a polymerizable double bond within the molecule, starting from the solid phase thereby forming a graft polymer chain. In case of chemically bonding the solid phase with a graft polymer chain, a functional group capable reacting with a functional group at a terminal end of the graft polymer chain is introduced into an inorganic material, and a graft polymer is chemically bonded thereto. Also in case of polymerizing a monomer, having a hydrophilic group and a polymerizable double bond within the molecule, starting from the solid phase thereby forming a graft polymer chain, a functional group, serving as a starting point for the polymerization of the compound having the double bond, is introduced into the inorganic material.

The graft polymer having a hydrophilic group and the monomer having a hydrophilic group and a polymerizable double bond within the molecule can preferably be the graft polymer having the hydrophilic group and the monomer having the hydrophilic group and the polymerizable double bond within the molecule, described above in the method of chemically bonding the solid phase of an organic material without a hydrophilic group and a graft polymer chain.

As another method for introducing a hydrophilic group into a solid phase of an inorganic material not having a hydrophilic group, a material having a hydrophilic group may be coated. A material to be used for coating is not particularly restricted as long as it has a hydrophilic group involved in the adsorption of nucleic acid, but is preferably a polymer of an organic material, in consideration of ease of operation. Such polymer can be, for example, polyhydroxyethylacrylic acid, polyhydroxyethylmethacrylic acid or a salt thereof; polyvinyl alcohol, polyvinylpyrrolidone, polyacrylic acid, polymethacrylic acid or a salt thereof; polyoxyethylene, acetylcellulose or a mixture of acetylcelluloses different in acetyl value.

It is also possible, after coating the solid phase of an inorganic material not having a hydrophilic group with acetylcellulose or a mixture of acetylcelluloses different in acetyl value, to conduct a saponification process on the acetylcellulose or the mixture of acetylcelluloses different in acetyl value thus coated. In such case, the saponification rate is preferably about 5 % or higher, more preferably about 10 % or higher.

The solid phase of an inorganic material not having a hydrophilic group may be prepared from a metal such as aluminum, glass, cement, ceramics such as porcelain, new ceramics, silicon or active charcoal.

A nucleic acid-adsorbing porous membrane advantageously employed as the solid phase in the invention is a material through which a solution can pass. A term "solution can pass through" means that, in case a pressure difference is generated between a space contacting a surface of the membrane and another space contacting the other surface of the membrane, a solution can pass through the interior of the membrane from the space of a higher pressure to the space of a lower pressure. Otherwise, it means that, when a centrifugal force is applied on the membrane, the solution can pass through the interior of the member in a direction of the centrifugal force.

The nucleic acid-adsorbing porous membrane preferably has a thickness of 10 to 500 µm, more preferably 50 to 250 µm. A smaller thickness is preferred in consideration of ease of washing.

The nucleic acid-adsorbing porous membrane may be symmetrical with respect to a front surface and a back surface thereof, but a porous membrane asymmetrical with respect to the front surface and the back surface can be preferably employed.

The nucleic acid-adsorbing porous membrane preferably has a minimum pore size of 0.22 µm or larger, more preferably 0.5 µm or larger. Also a preferred porous membrane has a ratio of a maximum pore size to a minimum pore size of 2 or larger, thereby providing a sufficient surface area for adsorbing nucleic acid and being not easily clogged. More preferably, the ratio of a maximum pore size to a minimum pore size is 5 or larger.

The nucleic acid-adsorbing porous membrane preferably has a void volume ratio of 50 to 95 %, more preferably 65 to 80 %, and preferably has a bubble point of 0.1 to 10 kgf/cm², more preferably 0.2 to 4 kgf/cm².

The nucleic acid-adsorbing porous membrane preferably has a pressure loss of 0.1 to 100 kPa, thereby providing a uniform pressure under an overpressure, more preferably 0.5 to 50 kPa. The pressure loss means a minimum pressure required for passing water per a membrane thickness of 100 µm.

The nucleic acid-adsorbing porous membrane preferably has a water permeation amount, when water is passed under a pressure of 1 kg/cm² at 25°C, of 1 to 5000 mL/min per 1 cm² of membrane, more preferably 5 to 1000 mL/min per 1 cm² of membrane under the same conditions.

The nucleic acid-adsorbing porous membrane preferably has a nucleic acid adsorption amount of 0.1 µg or higher per 1 mg of the porous membrane, more preferably 0.9 µg or higher per 1 mg of the porous membrane.

The nucleic acid-adsorbing porous membrane is preferably constituted of a cellulose derivative which, when a square porous membrane having a side of 5 mm is immersed in 5 mL of trifluoroacetic acid, does not dissolve in 1 hour but dissolves within 48 hours. Also preferred is a cellulose derivative which dissolves within 1 hour when a square porous membrane having a side of 5 mm is immersed in 5 mL of trifluoroacetic acid, but does not dissolve within 24 hours when immersed in 5 mL of dichloromethane. Among these, more preferred is a cellulose derivative which dissolves within 1 hour when a square porous membrane having a side of 5 mm is immersed in 5 mL of trifluoroacetic acid, but does not dissolve within 24 hours when immersed in 5 mL of dichloromethane.

When the sample solution containing the water-soluble organic solvent is passed through the nucleic acid-adsorbing porous membrane, the sample solution is preferably passed from a surface to the other surface thereof, in realizing a uniform contact of the solution with the membrane. When the sample solution containing the water-soluble organic solvent is passed through the nucleic acid-adsorbing porous membrane, the sample solution is preferably passed from a side thereof having a larger pore size to a side having a smaller pore size, because the clogging does not easily take place.

When the sample solution containing the water-soluble organic solvent is passed through the nucleic acid-adsorbing porous membrane, a flow rate is preferably 2 to 1500 µL/sec per 1 cm² of the membrane, in order to obtain an appropriate contact time of the solution with the porous membrane. An excessively short contact time of the solution with the porous membrane cannot provide a sufficient separating and purifying effect, while an excessively long contact time is undesirable in consideration of the operation efficiency. The flow rate is more preferably 5 to 700 µL/sec per 1 cm² of the membrane.

The nucleic acid-adsorbing porous membrane through which the used solution can pass, may be formed by a single membrane, but may also be formed by plural membranes. The plural nucleic acid-adsorbing porous membranes may be mutually same or different.

The plural nucleic acid-adsorbing porous membranes may be formed by a combination of a nucleic acid-adsorbing porous membrane of an inorganic material and a nucleic acid-adsorbing porous membrane of an organic material. For example, a combination of a glass filter and a regenerated cellulose porous membrane may be employed. Also the plural nucleic acid-adsorbing porous membranes may be formed by a combination of a nucleic acid-adsorbing porous membrane of an inorganic material and a nucleic acid non-adsorbing porous membrane of an organic material. For example, a combination of a glass filter and a nylon or polysulfone porous membrane may be employed.

A nucleic acid separation-purification cartridge, accommodating the aforementioned nucleic acid-adsorbing porous membrane through which a solution can pass, within a container having at least two openings, can be employed advantageously. Also a nucleic acid separation-purification cartridge, accommodating plural nucleic acid-adsorbing porous membranes through which a solution can pass, within a container having at least two openings, can be employed advantageously. In such case, the plural nucleic acid-adsorbing porous membranes, accommodated within the container having at least two openings, may be mutually same or different.

The nucleic acid separation-purification cartridge preferably does not contain, in the container having at least two openings, any member other than the nucleic acid-adsorbing porous membrane through which a solution can pass. The container may be formed by a plastic material such as polypropylene, polystyrene, polycarbonate or polyvinyl chloride. Also a biodegradable material can be employed advantageously. Also the container may be transparent or colored.

The nucleic acid separation-purification cartridge may be provided with means which identifies individual cartridge. Such means which identifies individual cartridge can be, for example, a bar code or a magnetic stripe.

Also the nucleic acid separation-purification cartridge may have a structure in which the nucleic acid-adsorbing porous membrane can be easily taken out from the container having at least two openings.
(3) A step of contacting a washing solution with the solid phase thereby washing the solid phase in a state where the nucleic acid is adsorbed thereon ("washing step"):

The washing step will be explained in the following.

The washing solution employed in the invention is an aqueous solution preferably containing a water-soluble organic solvent at a concentration of 50 mass% or less, more preferably at a concentration of 1 to 50 mass%. The washing solution is required to have a function of washing off an impurity which is present in the sample solution and is adsorbed, together with nucleic acid, on the nucleic acid-adsorbing membrane. For this reason, it preferably has such a composition that does not desorb nucleic acid but desorbs the impurity from the nucleic acid-adsorbing membrane.

The water-soluble organic solvent contained in the washing solution can be an alcohol, such as methanol, ethanol, isopropanol or an isomer thereof, or a butanol or an isomer thereof. More preferably, at least one selected from such alcohols is contained by 20 to 50 mass%, among which ethanol is most preferable.

The washing solution of the invention preferably contains further a water-soluble salt. The water-soluble salt is preferably a halide salt, and most preferably a chloride. Also the water-soluble salt is preferably formed by a monovalent or divalent cation, particularly preferably an alkali metal salt or an alkali earth metal salt, among which a sodium salt or a potassium salt is preferred and a sodium salt is most preferred.

The water-soluble salt, in case contained in the washing solution, has a concentration which is preferably equal to or higher than 10 mmol/L, and of which an upper limit, though not particularly restricted as long as the solubility of the impurity is not impaired, is preferably 1 mol/L or less and more preferably 0.1 mol/L or less. More preferably the water-soluble salt is sodium chloride which is further preferably contained by 20 mmol/L or higher.

The washing solution is further featured in being free from a chaotropic substance. It is thus rendered possible to reduce a possibility of contamination by a chaotropic substance in a recovering step after the washing step. As a contamination by a chaotropic substance in the recovering step often hinders an enzyme reaction such as a PCR reaction, the washing solution is ideally free from a chaotropic substance in consideration of subsequent enzyme reactions. Also as the chaotropic substance is corrosive and toxic, a process capable of dispensing with the chaotropic substance is extremely advantageous for the safety of the operator in the experimental operations.

The chaotropic substance means, as described above, urea, guanidine isothiocyanate, guanidine thiocyanate, sodium isocyanate, sodium iodide or potassium iodide.

In a washing step in a prior method for separating and purifying nucleic acid, the washing solution has a high wetting property to a container such as a cartridge and often remains in the container, whereby the washing solution causes a contamination in a recovering step subsequent to the washing step, thereby leading to a lowered purity of nucleic acid or a lowered reactivity in a subsequent step. Therefore, in case of conducting adsorption and desorption of nucleic acid in a container such as a cartridge, it is important that liquids employed for adsorption and washing, particularly the washing solution, do not remain in the cartridge after the washing so as not to affect the subsequent step.

Therefore, in order to minimize the washing solution remaining in the cartridge in the washing step and to prevent the washing solution from contaminating the recovering solution in the next step, the washing solution preferably has a surface tension less than 0.035 J/m². A lower surface tension improves the wetting property of the washing liquid on the cartridge, thereby reducing the remaining liquid amount.

An increased proportion of water may be employed for improving the washing efficiency, but elevates the surface tension of the washing solution, thereby increasing the remaining solution amount. In case the washing solution has a surface tension of 0.035 J/m² or higher, the remaining liquid amount can be suppressed by elevating the water repellency of the cartridge. With an increased water repellency of the cartridge, the solution forms liquid drops and flows down, thereby reducing the remaining liquid amount. The water repellency can be improved by coating the cartridge surface with a water repellent material such as silicone or by blending a water repellent material such as silicone at the cartridge molding, but such methods are not restrictive. Also the washing and recovering operations may be automated to conduct the operations in simple and prompt manner. The washing step may be conducted only once for a prompt process, but is preferably repeated plural times in case the purity is more important.

In the washing step, the washing solution is supplied by a tube, a pipette, an automatic injecting apparatus or supply means of an equivalent function to the nucleic acid separation-purification cartridge accommodating the nucleic acid-adsorbing porous membrane. The supplied washing solution is supplied from one of the openings of the nucleic acid separation-purification cartridge (the opening through which the sample solution is injected), and is passed through the nucleic acid-adsorbing porous membrane by pressurizing the interior of the nucleic acid separation-purification cartridge by a pressure generating apparatus (such as a squirt, a syringe, a pump or a power pipette) coupled with the aforementioned one opening, thereby being discharged from an opening different from the one opening. It is also possible to supply the washing solution from the one opening and discharge it from the same one opening. It is furthermore possible to supply and discharge the washing solution from an opening different from the opening of the nucleic acid separation-purification cartridge through which the sample solution is supplied. However a method of supplying the washing solution from the one opening of the nucleic acid separation-purification cartridge, causing it to pass through the nucleic acid-adsorbing porous membrane and discharging it from an opening different from the one opening, is preferred because of a superior washing efficiency. An amount of the washing solution in the washing step is preferably 2 µl/cm² or higher. Though a large solution amount can improve the washing effect, the amount is preferably 200 µl/cm² or less in order to maintain the efficiency of operation and to suppress a loss in the sample.

In the washing step, a flow rate of the washing solution in passing through the nucleic acid-adsorbing porous membrane is preferably 2 to 1500 µL/sec per a unit area (1 cm²) of the membrane, and more preferably 5 to 700 µL/sec. The washing operation can be conducted more sufficiently with a longer time under a lower flow rate, but the above-described range is selected because a prompter operation is also important in separation-purification of nucleic acid.

In the washing step, the washing solution preferably has a solution temperature of 4 to 70°C, and more preferably the room temperature. In the washing step, the washing operation may be conducted under an agitation by a mechanical vibration or an ultrasonic vibration applied to the nucleic acid separation-purification cartridge, or under a centrifugal force applied thereto.

In the invention, the washing step can be simplified by utilizing the nucleic acid-adsorbing porous membrane, more specifically (1) the step being conducted by a single passing of the washing solution through the nucleic acid-adsorbing porous membrane, (2) the step being executable at the room temperature, (3) the recovering solution being injectable into the cartridge immediately after the washing, and (4) the process being realizable with either one of the features (1), (2) and (3) or two or more thereof. This is because the thin nucleic acid-adsorbing porous membrane of the invention allows to dispense with a drying step, which is often required in a prior process for promptly removing an organic solvent contained in the washing solution.

In a prior nucleic acid separation-purification process, a washing solution in a washing step is often scattered and deposited elsewhere thereby causing a contamination in the sample. Such contamination can be suppressed by suitably designing shapes of the nucleic acid separation-purification cartridge accommodating a nucleic acid-adsorbing porous membrane in a container having two openings and of a waste liquor container.
(4) A step of contacting a recovering solution with the solid phase thereby desorbing the nucleic acid from the solid phase ("recovering step"):
   In the following a step of desorbing and recovering the nucleic acid from the solid phase will be explained.

In the recovering step, the recovering solution is supplied by a tube, a pipette, an automatic injecting apparatus or supply means of an equivalent function to the nucleic acid separation-purification cartridge accommodating the nucleic acid-adsorbing porous membrane. The recovering solution is supplied from one of the openings of the nucleic acid separation-purification cartridge (the opening through which the sample solution is injected), and is passed through the nucleic acid-adsorbing porous membrane by pressurizing the interior of the nucleic acid separation-purification cartridge by a pressure generating apparatus (such as a squirt, a syringe, a pump or a power pipette) coupled with the aforementioned one opening, thereby being discharged from an opening different from the one opening. It is also possible to supply the recovering solution from the one opening and discharge it from the same one opening. It is furthermore possible to supply and discharge the recovering solution from an opening different from the opening of the nucleic acid separation-purification cartridge through which the sample solution is supplied. However a method of supplying the recovering solution from the one opening of the nucleic acid separation-purification cartridge, causing it to pass through the nucleic acid-adsorbing porous membrane and discharging it from an opening different from the one opening, is preferred because of a superior recovering efficiency.

The nucleic acid may be desorbed by regulating a volume of the recovering solution, with respect to the volume of the sample solution containing the water-soluble organic solvent. An amount of the recovering solution, containing the recovered nucleic acid, depends on the amount of the involved analyte. An ordinary amount of the recovering solution is about several tens to several hundreds of microliters, but it may be changed within a range from 1 µl to several tends of milliliters in case of handling an extremely small amount of analyte or a separation-purification of a large amount of nucleic acid.

The recovering solution is preferably purified distilled water or a Tris/EDTA buffer. Also in case the recovered nucleic acid is used for a PCR (polymerase chain reaction), there may be employed a buffer to be used in the PCR reaction (for example aqueous solution with final concentrations of KCl: 50 mmol/L, Tris-HCl: 10 mmol/L and MgCl₂: 1.5 mmol/L).

The recovering solution preferably has a pH value within a range of 2 to 11, more preferably 5 to 9. The ionic strength and the salt concentration have a particular effect on the dissolution of the adsorbed nucleic acid. Preferably the recovering solution has an ionic strength of 290 mmol/L or less and a salt concentration of 90 mmol/L or less. These ranges improves a recovering rate of nucleic acid, thereby allowing to recover a larger amount of nucleic acid.

A concentrated nucleic acid-containing recovering solution can be obtained by reducing the volume of the recovering solution, in comparison with the volume of the sample solution containing the water-soluble organic solvent. A ratio (volume of recovering solution):
(volume of sample solution containing water-soluble organic solvent) may be preferably selected within a range of 1:100 to 99:100, more preferably within a rang of 1:10 to 9:10. In this manner, nucleic acid can be concentrated without a concentrating operation in a post-step of separation-purification of nucleic acid, and
there can be provided a method of obtaining a nucleic acid solution in which nucleic acid is more concentrated than in the original analyte.

It is also possible, as another method, by desorbing nucleic acid utilizing the recovering solution in a volume larger than the volume of the sample solution containing the water-soluble organic solvent, to obtain a recovering solution containing nucleic acid of a desired concentration, for example a recovering solution containing nucleic acid at a concentration suitable for a subsequent step (such as PCR). A ratio (volume of recovering solution) : (volume of sample solution containing water-soluble organic solvent) may be preferably selected within a range of 1:1 to 50:1, more preferably within a range of 1:1 to 5:1. In this manner, a cumbersome regulation of concentration after the separation-purification of nucleic acid. It is also possible to increase the nucleic acid recovery rate from the porous membrane, by employing the recovering solution of a sufficient amount.

Also a simple recovery of nucleic acid can be achieved by varying the temperature of the recovering solution according to the purpose. For example a nucleic acid desorption from the porous membrane with a recovering solution of a temperature of 0 to 10°C allows to suppress the function of nuclease thereby preventing decomposition of nucleic acid without any particular reagent or operation for avoiding an enzymatic decomposition, thus obtaining a nucleic acid solution in simple and efficient manner.

Also the recovering solution of a temperature of 10 to 35°C allows to conduct the recovery of nucleic acid at the room temperature and to desorb and purify nucleic acid without complex steps.

Also as another method, the recovering solution of a higher temperature for example of 35 to 70°C allows to conduct the desorption of nucleic acid from the porous membrane with a high recovery rate, in a simple process without involving complex operations.

The recovering solution is not particularly restricted in a number of injection and may be injected one or plural times. A simple and prompt separation-purification of nucleic acid is normally conducted with a single recovering operation, but the recovering solution may be injected plural times for example in case of recovering a large amount of nucleic acid.

In the recovering step, the recovering solution for nucleic acid may have a formulation usable in subsequent steps. The separated and purified nucleic acid is often amplified by a PCR (polymerase chain reaction) method. In such case, the solution of separated and purified nucleic acid has to be diluted with a buffer solution suitable for the PCR method. A buffer suitable the PCR method may be employed as the recovering solution in the recovering step of the invention, thereby enabling a simple and prompt transfer to the subsequent PCR step.

Also in the recovering step, the recovering solution for nucleic acid may further contain a stabilizing agent for preventing a decomposition of nucleic acid. Such stabilizing agent can be an antiseptic, an antimold agent or an inhibitor for the nuclease. An inhibitor for nuclease can be, for example, EDTA. In another embodiment, the stabilizing agent may be added in advance to a recovering container.

A recovering container to be used in the recovering step is not particularly restricted, and may be prepared with a material having no absorption at a wavelength of 260 nm. In such case, a concentration of the solution of recovered nucleic acid can be measured directly without requiring a transfer to another container. The material having no absorption at 260 nm can be, for example, a quartz glass, but such example is not restrictive.

Also a nucleic acid separation-purification cartridge to be employed in the above-described method of separating and purifying nucleic acid, and reagents to be used in the steps (1) to (4) may be provided as a kit.

The above-described steps of separating and purifying nucleic acid from the sample solution containing the water-soluble organic solvent, utilizing a nucleic acid separation-purification cartridge accommodating a nucleic acid-adsorbing porous membrane in a container having at least two openings and a pressure generating apparatus, are preferably conducted by an automatic apparatus capable of automatically conducting the steps. Such method allows not only to conduct the operations in a simpler and faster manner but also to obtain nucleic acid of a constant level without relying on the skill of the operator.

In the following, there will be explained an example of the automatic apparatus for automatically conducting the steps of separating and purifying nucleic acid from the sample solution containing the water-soluble organic solvent, utilizing a nucleic acid separation-purification cartridge accommodating a nucleic acid-adsorbing porous membrane in a container having at least two openings and a pressure generating apparatus, but the automatic apparatus is not limited to such example.

The automatic apparatus is a nucleic acid separation-purification apparatus for automatically conducting separation-purification operations by employing a nucleic acid separation-purification cartridge accommodating a nucleic acid-adsorbing porous membrane through which a solution can pass; injecting and pressurizing a sample solution containing a water-soluble organic solvent into the nucleic acid separation-purification cartridge thereby causing nucleic acid in the sample solution to be adsorbed in the nucleic acid-adsorbing porous membrane; separately injecting and pressurizing a washing solution in the nucleic acid separation-purification cartridge thereby removing an impurity; and separately injecting a recovering solution in the nucleic acid separation-purification cartridge thereby desorbing nucleic acid adsorbed on the nucleic acid-adsorbing porous membrane and recovering the nucleic acid together with the recovering solution, the apparatus being characterized in including a nucleic acid separation-purification cartridge; a mounting mechanism for supporting a waste liquor container for containing a residue of the sample solution containing the water-soluble organic solvent and a discharged washing solution, and a recovering container for containing the recovering solution containing nucleic acid; a pressurized air supply mechanism for introducing pressurized air into the nucleic acid separation-purification cartridge; and an injecting mechanism for separately injecting the washing solution and the recovering solution into the nucleic acid separation-purification cartridge.

### Examples

(1) Preparation of nucleic acid separation-purification cartridge

A nucleic acid separation-purification cartridge is prepared with an internal diameter of 7 mm and a portion for accommodating a nucleic acid-adsorbing porous membrane.
(2) A porous membrane, prepared by a saponification process on a triacetylcellulose porous membrane, is employed as the nucleic acid-adsorbing porous membrane and is accommodated in the accommodating portion of the nucleic acid separation-purification cartridge prepared in (1).
(3) Preparation of a stock solution of nucleic acid-solubilizing reagent, a washing solution, a recovering solution and a DNase solution

A nucleic acid-solubilizing reagent, a washing solution, a recovering solution and a DNase solution are prepared with following formulations:

| (Stock solution of nucleic acid-solubilizing reageant) | |
|---|---|
| Guanidine hydrochloride | 528.4 g |

| (manufactured by Wako Pure Chemical Ind. Ltd.) | |
|---|---|
| Olfin AK-02 | 5.59 g |

| (manufactured by Nisshin Chemical.Co.) | |
|---|---|
| Leodol TWS-120V (manufactured by Kao Corp) | 32.95 g |

| | |
|---|---|
| Ethanol | 64.8 g |

| (manufactured by Wako Pure Chemical Ind. Ltd.) | |
|---|---|
| Cetyl trimethyl ammonium bromide (CTAB) | 22.3 g |

| (manufactured by Wako Pure Chemical Ind. Ltd.) | |
|---|---|
| Distilled water | 575.3 g |

| (Washing solution) | |
|---|---|
| Distilled water | 466.8 g |
| 1 mol/L trishydrochloric acid (pH: 7.5) | 7.04 g |

| (manufactured by Wako Pure Chemical Ind. Ltd.) | |
|---|---|
| Sodium chloride | 3.95 g |

| (manufactured by Wako Pure Chemical Ind. Ltd.) | |
|---|---|
| (Recovering solution) | |
| Tris-HCl (pH: 6.5) | 1 mmol/L |

| (DNase solution 1) | |
|---|---|
| DNase (manufactured by Promega Inc.) | 360 µl |
| DNase buffer | 72 µl |

| (manufactured by Promega Inc., packed with DNase) | |
|---|---|
| Distilled water | 288 µl |

| (DNase solution 2) | |
|---|---|
| DNase (manufactured by Qiagen Inc.) | 11.25 µl |
| DNase buffer | 315 µl |

| (manufactured by Qiagen Inc., packed with DNase) | |
|---|---|
| Distilled water | 33.75 µl |

(4) Nucleic acid separation-purification operations

### [Example 1]

Hela cells were cultured as adherent cells in an on-dish method to obtain a sample solution containing a water-soluble organic solvent of Example 1 in the following manner.

On a 6-hole cell culture plate, Hela cells were cultured in a culture solution (MEM - 10% bovine fetus serum) at 37°C in the presence of 5% CO₂. A number of cells cultured at the same time was measured as 3.12 x 10⁶ per hole. The culture solution was removed from a hole of the cell culture plate and the stock solution of nucleic acid-solubilizing reagent was added to obtain a cell solution. The cell solution was agitated by pipetting and recovered in another container.

516 µl of the stock solution of nucleic acid-solubilizing reagent were added with 4 µl of 2-mercaptoethanol to obtain a lysis solution, which was entirely added to the cell-containing container and agitated for 1 minute by a vortex mixer. Then 100 µl of 99.5 vol.% ethanol were added and agitated for 5 seconds with a vortex mixer. Then 180 µl of 99.5 vol.% ethanol were added to obtain a final ethanol concentration of 35 mass%, and the mixture was agitated for 5 seconds with a vortex mixer.

Thus obtained sample solution of Example 1, containing the water-soluble organic solvent, was injected in one of the openings of the nucleic acid separation-purification cartridge accommodating the nucleic acid-adsorbing porous membrane as prepared in (1) and (2), then the pressure generating apparatus was coupled with the one opening to pressurize the interior of the nucleic acid separation-purification cartridge thereby causing the injected sample solution containing the water-soluble organic solvent to pass through the nucleic acid-adsorbing porous membrane and thereby contacting nucleic acid therewith, and to discharge the sample solution from the other opening of the nucleic acid separation-purification cartridge. Subsequently the pressure generating apparatus was detached, then 500 µl of the washing solution containing ethanol at a concentration of 30.viol.% were injected into the one opening of the nucleic acid separation-purification cartridge, and the pressure generating apparatus was coupled with the one opening to pressurize the interior of the nucleic acid separation-purification cartridge thereby causing the injected washing solution to pass through the nucleic acid-adsorbing porous membrane and discharging the washing solution from the other opening. These operations were repeated three times in a similar manner. Then the pressure generating apparatus ways detached, 100 µl of the recovering solution were injected into the one opening of the nucleic acid separation purification cartridge, and the pressure generating apparatus was coupled with the one opening thereof to pressurize the interior thereof thereby causing the injected recovering solution to pass through the nucleic acid-adsorbing porous membrane and discharging the recovering solution from the other opening, and the solution was recovered.

### [Comparative Example 1]

Hela cells were cultured as adherent cells in an on-dish method to obtain a sample solution containing a water-soluble organic solvent of Comparative Example 1 in the following manner.

On a 6-hole cell culture plate, Hela cells where cultured in a culture solution (MEM - 10% bovine fetus strum) at 37°C in the presence of 5% C₂. A number of cells cultured at the same time was measured as 3.12 x 10⁶ per hole. The culture solution was removed from a hole of the cell culture plate and the stock solution of nucleic acid-solubilizing reagent was added to obtain a cell solution. The cell solution was agitated by pipetting and recovered.in another container.

350 µl of the stock solution of nucleic acid-solubilizing reagent were added with 3.5 µl of 2-mercaptoethanol to obtain a lysis solution, which was entirely added to the cell-containing container and agitated for 1 minute by a vortex mixer. Then 350 µl of 70 vol.% ethanol were added and agitated for 5 seconds with a vortex mixer.

The obtained solution, after agitation, was injected in one of the openings of the nucleic acid separation-purification cartridge accommodating the nucleic acid-adsorbing porous membrane as prepared in (1) and (2), then the pressure generating apparatus was coupled with the one opening to pressurize the interior of the nucleic acid separation-purification cartridge thereby causing the injected nucleic acid mixture solution to pass through the nucleic acid-adsorbing porous membrane and thereby contacting the mixture solution with the membrane, and to discharge the solution from the other opening of the nucleic acid separation-purification cartridge. Then the pressure generating apparatus was detached, 500 µl of the washing solution containing ethanol at a concentration of 30 vol.% were injected into the one opening of the nucleic acid separation-purification cartridge, and the pressure generating apparatus was coupled with the one opening to pressurize the interior thereof thereby causing the injected washing solution to pass through the nucleic acid-adsorbing porous membrane and discharging the washing solution from the other opening. These operations were repeated three times in a similar manner. Then the pressure generating apparatus was detached, 100 µl of the recovering solution were injected into the one opening of the nucleic acid separation-purification cartridge, and the pressure generating apparatus was coupled with the one opening thereof to pressurize the interior thereof thereby causing the injected recovering solution to pass through the nucleic acid-adsorbing porous membrane and discharging the recovering solution from the other opening, and the solution was recovered.

### [Example 2]

A culture solution of human acute promyelocytic leukemia cells (HL60) was prepared. It was so regulated as to obtain a cell.count of 3 x 10⁶ and the cells were washed with PBS free from Ca²⁺ and Mg²⁺. A centrifuging was conducted with a swinging rotor under conditions of 4°C, 300 G and 5 minutes, to pelletize the floating cells, then a supernatant solution was removed and the cells were re-suspended by a tapping. 516 µl of the stock solution of nucleic acid-solubilizing reagent were added with 4 µl of 2-mercaptoethanol to obtain a lysis solution, which was entirely added to the cell-containing container and agitated for 1 minute by a vortex mixer. Then 100 µl of 99.5 vol.% ethanol were added and agitated for 5 seconds with a vortex mixer. Then 180 µl of 99.5 vol.% ethanol were added to obtain a final ethanol concentration of 35 mass%, and the mixture was agitated for 5 seconds with a vortex mixer. A period from the start of cell washing with PBS to the end of agitation was 10 minutes.

Thus obtained sample solution of Example 2, containing the water-soluble organic solvent, was injected in one of the openings of the nucleic acid separation-purification cartridge accommodating the nucleic acid-adsorbing porous membrane as prepared in (1) and (2), then the pressure generating apparatus was coupled with the one opening to pressurize the interior of the nucleic acid separation-purification cartridge thereby causing the injected sample solution containing the water-soluble organic solvent to pass through the nucleic acid-adsorbing porous membrane and hereby contacting the sample solution therewith, and to discharge the sample solution from the other opening of the nucleic acid separation-purification cartridge. Subsequently the pressure generating apparatus was detached, then 500 µl of the washing solution containing ethanol at a concentration of 30 vol.%.were injected into the one opening of the nucleic acid separation-purification cartridge, and the pressure generating apparatus was coupled with the one opening to pressurize the interior of the nucleic acid separation-purification cartridge thereby causing the injected washing solution to pass through the nucleic acid-adsorbing porous membrane and discharging the washing solution from the other opening. Subsequently the pressure generating apparatus was detached, then 40 µl of a DNase solution were placed on the membrane in the nucleic acid separation-purification cartridge through the one opening thereof, then after a standing for 5 minutes, 500 µl of the washing solution containing ethanol at a concentration of 30 vol.% were injected into the one opening of the nucleic acid separation-purification cartridge, and the pressure generating apparatus was coupled with the one opening to pressurize the interior of the nucleic acid separation-purification cartridge thereby causing the injected washing solution to pass through the nucleic acid-adsorbing porous membrane and discharging the washing solution from the other opening. These operations were repeated once again in a similar manner. Subsequently, the pressure generating apparatus was detached, then 100 µl of the recovering solution were injected into the one opening of the nucleic acid separation-purification cartridge, and the pressure generating apparatus was coupled with the one opening thereof to pressurize the interior thereof thereby causing the injected recovering solution to pass through the nucleic acid-adsorbing porous membrane and discharging the recovering solution from the other opening, and the solution was recovered. Example 2 was conducted four times with the DNase solution 1 as the DNase solution, and four times with the DNase solution 2 as the DNase solution.

### [Comparative Example 2]

A culture solution of human acute promyelocytic leukemia cells (HL60) was prepared. It was so regulated as to obtain a cell count of 3 x 10⁶ and the cells were washed with PBS free from Ca²⁺ and Mg²⁺. A centrifuging was conducted with a swinging rotor under conditions of 4°C, 300 G and 5 minutes, to pelletize the floating cells, then a supernatant solution was removed and the cells were suspended by a tapping. 350 µl of the stock solution of nucleic acid-solubilizing reagent were added with 3.5 µl of 2-mercaptoethanol to obtain a lysis solution, which was entirely added to the cell-containing container and agitated for 1 minute by a vortex mixer. Then 350 µl of 70 vol.% ethanol were added and agitated for 5 seconds with a vortex mixer. A period from the start of cell washing with PBS to the end of agitation was 10 minutes.

The obtained solution, after agitation, was injected in one of the openings of the nucleic acid separation purification cartridge accommodating the nucleic acid-adsorbing porous membrane as prepared in (1) and (2), then the pressure generating apparatus was coupled with the one opening to pressurize the interior of the nucleic acid separation-purification cartridge thereby causing the injected nucleic acid mixture solution to pass through the nucleic acid-adsorbing porous membrane and contacting the solution with the membrane, and to discharge the solution from the other opening of the nucleic acid separation-purification cartridge. Subsequently the pressure generating apparatus was detached, then 500 µl of the washing solution containing ethanol at a concentration of 30 vol.% were injected into the one opening of the nucleic acid separation-purification cartridge, and the pressure generating apparatus was coupled with the one opening to pressurize the interior of the nucleic acid separation-purification cartridge thereby causing the injected washing solution to pass through the nucleic acid-adsorbing porous membrane and discharging the washing solution from the other opening. Subsequently the pressure generating apparatus was detached, then 40 µl of a DNase solution were placed on the membrane in the nucleic acid separation-purification cartridge through the one opening thereof, then after a standing for 5 minutes, 500 µl of the washing solution containing ethanol at a concentration of 30 vol.% were injected into the one opening of the nucleic acid separation-purification cartridge, and the pressure generating apparatus was coupled with the one opening to pressurize the interior of the nucleic acid separation-purification cartridge thereby causing the injected washing solution to pass through the nucleic acid-adsorbing porous membrane and discharging the washing solution from the other opening. These operations were repeated once again in a similar manner. Subsequently, the pressure generating apparatus was detached, then 100 µl of the recovering solution were injected into the one opening of the nucleic acid separation-purification cartridge, and the pressure generating apparatus was coupled with the one opening of the nucleic acid separation-purification cartridge to pressurize the interior thereof thereby causing the injected recovering solution to pass through the nucleic acid-adsorbing porous membrane and discharging the recovering solution from the other opening, and the solution was recovered. Comparative Example 2 was conducted four times with the DNase solution 1 as the DNase solution, and four times with the DNase solution 2 as the DNase solution.

### [Example 3]

A culture solution of human acute promyelocytic leukemia cells (HL60) was prepared. It was so regulated as to obtain a cell count of 5 x 10⁶ and the cells were washed with PBS free from Ca²⁺ and Mg²⁺. A centrifuging was conducted with a swinging rotor under conditions of 4°C, 300 G and 5 minutes, to pelletize the floating cells, then a supernatant solution was removed and the cells were re-suspended by a tapping. 516 µl of the stock solution of nucleic acid-solubilizing reagent were added with 4 µl of 2-mercaptoethanol to obtain a lysis solution, which was entirely added to the cell-containing container and agitated for 1 minute by a vortex mixer. Then 100 µl of 99.5·vol.% ethanol were added and agitated for 5 seconds with a vortex mixer. Then 180 µl of 99.5 vol.% ethanol were added to obtain a final ethanol concentration of 35 mass%, and the mixture was agitated for 5 seconds with a vortex mixer.

Thus obtained sample solution of Example 3, containing the water-soluble organic solvent, was injected in one of the openings of the nucleic acid separation-purification cartridge accommodating the nucleic acid-adsorbing porous membrane as prepared in (1) and (2), then the pressure generating apparatus was coupled with the one opening to pressurize the interior of the nucleic acid separation-purification cartridge thereby causing the injected sample solution containing the water-soluble organic solvent to pass through the nucleic acid-adsorbing porous membrane and thereby contacting the sample solution therewith, and to discharge the sample solution from the other opening of the nucleic acid separation-purification cartridge. Subsequently the pressure generating apparatus was detached, then 500 µl of the washing solution containing ethanol at a concentration of 30 vol.% were injected into the one opening of the nucleic acid separation-purification cartridge, and the pressure generating apparatus was coupled with the one opening to pressurize the interior of the nucleic acid separation-purification cartridge thereby causing the injected washing solution to pass through the nucleic acid-adsorbing porous membrane and discharging the washing solution from the other opening. These operations were repeated three times in a similar manner. Subsequently, the pressure generating apparatus was detached, then 100 µl of the recovering solution were injected into the one opening of the nucleic acid separation-purification cartridge, and the pressure generating apparatus was coupled with the one opening thereof to pressurize the interior thereof thereby causing the injected recovering solution to pass through the nucleic acid-adsorbing porous membrane and discharging the recovering solution from the other opening, and the solution was recovered.

### [Comparative Example 3]

A culture solution of human acute promyelocytic leukemia cells (HL60) was prepared. It was so regulated as to obtain a cell count of 5 x 10⁶ and the cells were washed with PBS free from Ca²⁺ and Mg²⁺. A centrifuging was conducted with a swinging rotor under conditions of 4°C, 300 G and 5 minutes, to pelletize the floating cells, then a supernatant solution was removed and the cells were re-suspended by a tapping. 516 µl of the stock solution of nucleic acid-solubilizing reagent were added with 4 µl of 2-mercaptoethanol to obtain a lysis solution, which was entirely added to the cell-containing container and agitated for 1 minute by a vortex mixer. Then 280 µl of 99.5 vol.% ethanol were added and agitated for 5 seconds with a vortex mixer. Then agitation was conducted for 5 seconds with a vortex mixer.

The obtained solution, after agitation, was injected in one of the openings of the nucleic acid separation-purification cartridge accommodating the nucleic acid-adsorbing porous membrane as prepared in (1) and (2), then the pressure generating apparatus was coupled with the one opening to pressurize the interior of the nucleic acid separation-purification cartridge thereby causing the injected sample solution containing the water-soluble organic solvent to pass through the nucleic acid-adsorbing porous membrane and thereby contacting the solution therewith, and to discharge the sample solution from the other opening of the nucleic acid separation-purification cartridge. Subsequently the pressure generating apparatus was detached, then 500µl of the washing solution containing ethanol at a concentration of 30 vol.% were injected into the one opening of the nucleic acid separation-purification cartridge, and the pressure generating apparatus was coupled with the one opening to pressurize the interior of the nucleic acid separation-purification cartridge thereby causing the injected washing solution to pass through the nucleic acid-adsorbing porous membrane and discharging the washing solution from the other opening. These operations were repeated three times in a similar manner. Subsequently, the pressure generating apparatus was detached, then 100 µl of the recovering solution were injected into the one opening of the nucleic acid separation-purification cartridge, and the pressure generating apparatus was coupled with the one opening thereof to pressurize the interior thereof thereby causing the injected recovering solution to pass through the nucleic acid-adsorbing porous membrane and discharging the recovering solution from the other opening, and the solution was recovered.

Table 1 summarizes amounts of the lysis solution, the dissolving stock solution (stock solution of nucleic acid-solubilizing reagent) and the solution containing the water-soluble organic solvent, employed in each example.

**Table 1**

| | lysis solution | | first ethanol solution | | second ethanol solution |
|---|---|---|---|---|---|
| | dissolving stock solution | 2-mercaptoethanol | 70 vol.% ethanol | 99.5 vol.% ethanol | 99.5 vol.% ethanol |
| Example 1 | 516 µl | 4 µl | - | 100 µl | 180 µl |
| Comparative Example 1 | 350 µl | 3.5 µl | 350 µl | - | - |
| Example 2 | 516 µl | 4 µl | - | 100 µl | 180 µl |
| Comparative Example 2 | 350 µl | 3.5 µl | 350 µl | - | - |
| Example 3 | 516 µl | 4 µl | - | 100 µl | 180 µl |
| Comparative Example 3 | 516 µl | 4 µl | - | 280 µl | - |

(5) Membrane-passing time of lysis solution and amount of recovered nucleic acid

In each example, an amount of nucleic acid, in the recovering solution containing the obtained nucleic acid, was determined by an absorbance at 230 nm. Table 2 summarizes the concentration of nucleic acid in the obtained recovering solution, and a measured membrane-passing time required for the lysis solution.

**Table 2**

| | recovered nucleic acid | membrane passing/no passing of lysis solution (average passing time) |
|---|---|---|
| Comparative Example 1 | - | no passing in 2 trials |
| Example 1 | 53.5 µg | 2 passings in 2 trials (48.0 sec) |
| Comparative Example 2 | 28. 1 µg | 8 passings in 8 trials (61.5 sec) |
| Example 2 | 34.9 µg | 2 passings in 2 trials (58.9 sec) |
| Comparative Example 3 | - | no passing in 2 trials |
| Example 3 | 492.0 µg | 2 passings in 2 trials (61.0 sec) |

Comparisons of Example 1 and Comparative Example 1 and of Example 2 and Comparative Example 2 in Tables 1 and 2 indicate that the membrane-passing time of the lysis solution became shorter in case of employing the lysis solution of 520 µl, also adding ethanol with a concentration of 99.5 vol.% and an amount of 280 µl and adding it in two portions. These results indicate that the method of the present invention allows to process a larger amount of cells. Stated differently, a processible upper limit of the biomaterial can be elevated by employing ethanol of a higher concentration, increasing the amount of the lysis solution and adding ethanol in two portions. Also a comparison of Example 3 and Comparative Example 3 indicates that addition of ethanol of 99.5 vol.% in two portions increases the processible cell number in comparison with addition at a time.

### Industrial Applicability

The present invention provides, in the method of separating and purifying nucleic acid by adsorbing nucleic acid in a biomaterial on a surface of a solid phase and, after washing and the like, desorbing nucleic acid, a method capable of processing a larger amount of biomaterial without prolonging a time for obtaining a solution for nucleic acid adsorption on the solid phase.

The present invention also allows to selectively recover nucleic acid from a biomaterial in more inexpensive and easier manner, utilizing a porous membrane showing an excellent separating efficiency and a satisfactory washing efficiency, enabling a simple and prompt use, being adapted for automation and compactification and mass producible with a substantially identical separating property.

## Claims

1. A method for separating and purifying a nucleic acid comprising steps of:
(1) adding a lysis solution to a biomaterial to prepare a sample solution containing a nucleic acid, and
adding a water-soluble organic solvent or a solution containing a water-soluble organic solvent to the sample solution thereby preparing a sample solution containing the water-soluble organic solvent;
(2) contacting the sample solution containing the water-soluble organic solvent with a solid phase thereby adsorbing the nucleic acid on the solid phase;
(3) contacting a washing solution with the solid phase thereby washing the solid phase in a state where the nucleic acid is adsorbed on the solid phase; and
(4) contacting a recovering solution with the solid phase thereby desorbing the nucleic acid from the solid phase,
wherein, in the step (1), the water-soluble organic solvent or the solution containing the water-soluble organic solvent is added separately in at least two batches,
wherein the amount of first addition is 5 to 90 vol.% of a total addition amount, and
wherein the step (1) further comprises:
after adding the water-soluble organic solvent or the solution containing the water-soluble organic solvent at least once to the sample solution, agitating the sample solution by an operation including at least one of a shaking, an inverting and a rotating movement; and further adding the water-soluble organic solvent or the solution containing th water-soluble organic solvent at least once to the sample solution after the agitation.

2. The method for separating and purifying a nucleic acid according to claim 1,
wherein the step (1) comprises:
after adding the water-soluble organic solvent or the solution containing the water-soluble organic solvent at least once to the sample solution, agitating the sample solution by an operation including at least a suction and a discharge of the solution, and
further adding the water-soluble organic solvent or the solution containing the water-soluble organic solvent at least once to the sample solution after the agitation,
whereby in case of conducting both the operation including at least one of a shaking, an inverting and a rotating movement and the operation including at least a suction and a discharge of the solution, these operations may be conducted in an arbitrary order.

3. The method for separating and purifying a nucleic acid according to any of claims 1 or 2,
wherein, in the step (1), the sample solution containing the water-soluble organic solvent has a concentration of the water-soluble organic solvent within a range of from 5 to 90 mass%.

4. The method for separating and purifying a nucleic acid according to any of claims 1 or 2,
wherein, in the step (1), the sample solution containing the water-soluble organic solvent has a concentration of the water-soluble organic solvent within a range of from 10 to 60 mass%.

5. The method for separating and purifying a nucleic acid according to any of claims 1 or 2,
wherein, in the step (1), the sample solution containing the water-soluble organic solvent has a concentration of the water-soluble organic solvent within a range of from 20 to 40 mass%.

6. The method for separating and purifying a nucleic acid according to any of claims 1 to 5,
wherein the solid phase is a porous membrane comprising an organic polymer that adsorbs a nucleic acid by an interaction substantially not involving an ionic bonding.

7. The method for separating and purifying a nucleic acid according to claim 6,
wherein the organic polymer has a hydroxyl group.

8. The method for separating and purifying a nucleic acid according to claim 6 or 7,
wherein the porous membrane comprises an organic material obtained by saponification of a mixture of acetylcelluloses different in acetyl value.

9. The method for separating and purifying a nucleic acid according to any of claims 6 to 8,
wherein the porous membrane has a front surface and a back surface asymmetrical with each other.

10. The method for separating and purifying a nucleic acid according to any of claims 1 to 9,
wherein the lysis solution is a nucleic acid-solubilizing reagent.

11. The method for separating and purifying a nucleic acid according to any of claims 1 to 10,
wherein the biomaterial is an animal tissue.

12. The method for separating and purifying a nucleic acid according to claim 10,
wherein the nucleic acid-solubilizing reagent comprises at least one selected from the group consisting
of a chaotropic salt, a nucleic acid-stabilizing agent, a surfactant, a buffer and a defoaming agent.

13. The method for separating and purifying a nucleic acid according to claim 12,
wherein the chaotropic salt comprises at least one selected from the group consisting of guanidine hydrochloride and guanidine thiocyanate.

14. The method for separating and purifying a nucleic acid according to any of claims 1 to 13,
wherein the water-soluble organic solvent is at least one selected from the group consisting of methanol, ethanol, propanol or an isomer thereof and butanol or an isomer thereof.

15. The method for separating and purifying a nucleic acid according to any of claims 1 to 14,
wherein the washing solution comprises at least one selected from the group consisting of methanol, ethanol, propanol or an isomer thereof and butanol or an isomer thereof, in an amount of from 20 to 50 mass.

16. The method for separating and purifying a nucleic acid according to any of claims 1 to 15,
wherein the washing solution is a solution comprising a chloride salt in an amount of from 10 mmol/L to 1 mol/L.

17. The method for separating and purifying a nucleic acid according to any of claims 8 to 16,
wherein, in the steps (2), (3) and (4), passing of the sample solution containing the water-soluble organic solvent, the washing solution or the recovering solution through the porous membrane is conducted by utilizing: a nucleic acid separating-purifying cartridge that receives the porous membrane which a solution can pass through in an inside of a container having at least two openings; and a pressure generating apparatus that is a pump detachably mountable on one of the at least two openings of the nucleic acid separating-purifying cartridge.

## Patentansprüche

1. Verfahren zur Abtrennung und Reinigung einer Nucleinsäure, umfassend die Schritte:
(1) Zugeben einer Lyselösung zu einem Biomaterial, um eine Probenlösung herzustellen, die eine Nucleinsäure enthält, und
Zusetzen eines wasserlöslichen organischen Lösungsmittels oder einer Lösung, die ein wasserlösliches organisches Lösungsmittel enthält, zu der Probenlösung, um dadurch eine Probenlösung herzustellen, die das wasserlösliche organische Lösungsmittel enthält;
(2) Inkontaktbringen der Probenlösung, die das wasserlösliche organische Lösungsmittel enthält, mit einer festen Phase, um dadurch die Nucleinsäure an der festen Phase zu adsorbieren;
(3) Inkontaktbringen einer Waschlösung mit der festen Phase, um dadurch die feste Phase in einem Zustand, in dem die Nucleinsäure an der festen Phase adsorbiert ist, zu waschen, und
(4) Inkontaktbringen einer Wiedergewinnungslösung mit der festen Phase, um dadurch die Nucleinsäure von der festen Phase zu desorbieren,
wobei in Schritt (1) das wasserlösliche organische Lösungsmittel oder die Lösung, die das wasserlösliche organische Lösungsmittel enthält, getrennt in wenigstens zwei Chargen zugesetzt wird,
wobei die Menge der ersten Zugabe 5 bis 90 Vol.-% einer gesamten Zugabemenge ist und wobei der Schritt (1) außerdem umfasst:
nach Zugeben des wasserlöslichen organischen Lösungsmittels oder der Lösung, die das wasserlösliche organische Lösungsmittel enthält, wenigstens ein Mal zu der Probenlösung, Bewegen der Probenlösung durch einen Arbeitsgang, der wenigstens eines von einem Schütteln, einem Umdrehen und einer Drehbewegung umfasst, und
außerdem zugeben des wasserlöslichen organischen Lösungsmittels oder der Lösung, die das wasserlösliche organische Lösungsmittel enthält, wenigstens ein Mal zu der Probenlösung nach der Bewegung.

2. Verfahren zur Abtrennung und Reinigung einer Nucleinsäure gemäß Anspruch 1, wobei der Schritt (1) umfasst:
nach Zugeben des wasserlöslichen organischen Lösungsmittels oder der Lösung, die das wasserlösliche organische Lösungsmittel enthält, wenigstens ein Mal zu der Probenlösung, Bewegen der Probenlösung durch einen Vorgang, der wenigstens ein Saugen und ein Austragen der Lösung umfasst, und
außerdem Zugeben des wasserlöslichen organischen Lösungsmittels oder der Lösung, die das wasserlösliche organische Lösungsmittel enthält, wenigstens ein Mal zu der Probenlösung nach dem Bewegen,
wodurch im Fall des Durchführens von beidem, dem Vorgang, der wenigstens eines von einem Schütteln, einem Umdrehen und einer Drehbewegung umfasst, und dem Vorgang, der wenigstens ein Saugen und ein Austragen der Lösung umfasst, diese Vorgänge in beliebiger Reihenfolge durchgeführt werden können.

3. Verfahren zur Abtrennung und Reinigung einer Nucleinsäure gemäß Anspruch 1 oder 2,
wobei in Schritt (1) die Probenlösung, die das wasserlösliche organische Lösungsmittel enthält, eine Konzentration des wasserlöslichen organischen Lösungsmittels innerhalb eines Bereichs von 5 bis 90 Massen% hat.

4. Verfahren zur Abtrennung und Reinigung einer Nucleinsäure gemäß Anspruch 1 oder 2,
wobei in Schritt (1) die Probenlösung, die das wasserlösliche organische Lösungsmittel enthält, eine Konzentration des wasserlöslichen organischen Lösungsmittels innerhalb eines Bereichs von 10 bis 60 Massen% hat.

5. Verfahren zur Abtrennung und Reinigung einer Nucleinsäure gemäß Anspruch 1 oder 2,
wobei in Schritt (1) die Probenlösung, die das wasserlösliche organische Lösungsmittel enthält, eine Konzentration des wasserlöslichen organischen Lösungsmittels innerhalb eines Bereichs von 20 bis 40 Massen% hat.

6. Verfahren zur Abtrennung und Reinigung einer Nucleinsäure gemäß einem der Ansprüche 1 bis 5,
wobei die feste Phase eine poröse Membran ist, die ein organisches Polymer umfasst, welches eine Nucleinsäure durch eine Wechselwirkung adsorbiert, die im Wesentlichen keine ionische Bindung involviert.

7. Verfahren zur Abtrennung und Reinigung einer Nucleinsäure gemäß Anspruch 6,
wobei das organische Polymer eine Hydroxylgruppe hat.

8. Verfahren zur Abtrennung und Reinigung einer Nucleinsäure gemäß Anspruch 6 oder 7,
wobei die poröse Membran ein organisches Material umfasst, das durch Verseifung eines Gemisches von Acetylcellulosen, die sich im Acetylwert unterscheiden, erhalten wird.

9. Verfahren zur Abtrennung und Reinigung einer Nucleinsäure gemäß einem der Ansprüche 6 bis 8,
wobei die poröse Membran eine vordere Oberfläche und eine rückseitige Oberfläche hat, die asymmetrisch zueinander sind.

10. Verfahren zur Abtrennung und Reinigung einer Nucleinsäure gemäß einem der Ansprüche 1 bis 9,
wobei die Lyselösung ein Nucleinsäure-solubilisierendes Reagens ist.

11. Verfahren zur Abtrennung und Reinigung einer Nucleinsäure gemäß einem der Ansprüche 1 bis 10,
wobei das Biomaterial ein Tiergewebe ist.

12. Verfahren zur Abtrennung und Reinigung einer Nucleinsäure gemäß Anspruch 10, wobei das Nucleinsäure-solubilisierende Reagens wenigstens eines umfasst, das aus der Gruppe, bestehend aus einem chaotropen Salz, einem Nucleinsäurestabilisierenden Mittel, einem Surfactant, einem Puffer und einem Entschäumungsmittel, ausgewählt wird.

13. Verfahren zur Abtrennung und Reinigung einer Nucleinsäure gemäß Anspruch 12, wobei das chaotrope Salz wenigstens eines umfasst, das aus der Gruppe, bestehend aus Guanidinhydrochlorid und Guanidinthiocyanat, ausgewählt wird.

14. Verfahren zur Abtrennung und Reinigung einer Nucleinsäure gemäß einem der Ansprüche 1 bis 13, wobei das wasserlösliche organische Lösungsmittel wenigstens eines ist, das aus der Gruppe, bestehend aus Methanol, Ethanol, Propanol oder einem Isomer davon und Butanol oder einem Isomer davon, ausgewählt wird.

15. Verfahren zur Abtrennung und Reinigung einer Nucleinsäure gemäß einem der Ansprüche 1 bis 14, wobei die Waschlösung wenigstens eines, ausgewählt aus der Gruppe, bestehend aus Methanol, Ethanol, Propanol oder einem Isomer davon und Butanol oder einem Isomer davon, in einer Menge von 20 bis 50 Massen% umfasst.

16. Verfahren zur Abtrennung und Reinigung einer Nucleinsäure gemäß einem der Ansprüche 1 bis 15, wobei die Waschlösung eine Lösung ist, die ein Chloridsalz in einer Menge von 10 mmol/L bis 1 mol/L umfasst.

17. Verfahren zur Abtrennung und Reinigung einer Nucleinsäure gemäß einem der Ansprüche 8 bis 16, wobei in den Schritten (2), (3) und (4) ein Durchleiten der Probenlösung, die das wasserlösliche organische Lösungsmittel enthält, der Waschlösung oder der Wiedergewinnungslösung durch die poröse Membran durchgeführt wird, indem verwendet wird: eine Nucleinsäure-Abtrennungs-Reinigungs-Kartusche, die die poröse Membran aufnimmt, durch welche eine Lösung im Inneren eines Behälters, der wenigstens zwei Öffnungen hat, gehen kann, und eine Druck erzeugende Apparatur, die eine Pumpe ist, die ablösbar an einer der wenigstens zwei Öffnungen der Nucleinsäure-Abtrennungs-Reinigungs-Kartusche montierbar ist.

## Revendications

1. Procédé pour séparer et purifier un acide nucléique, comprenant les étapes consistant à :
(1) ajouter une solution de lyse à un biomatériau afin de préparer une solution d'échantillon contenant un acide nucléique, et ajouter un solvant organique hydrosoluble ou une solution contenant un solvant organique hydrosoluble à la solution d'échantillon pour ainsi préparer une solution d'échantillon contenant le solvant organique hydrosoluble ;
(2) mettre en contact la solution d'échantillon contenant le solvant organique hydrosoluble avec une phase solide pour ainsi adsorber l'acide nucléique sur la phase solide ;
(3) mettre en contact une solution de lavage avec la phase solide pour ainsi laver la phase solide dans un état où l'acide nucléique est adsorbé sur la phase solide ; et
(4) mettre en contact une solution de récupération avec la phase solide pour ainsi désorber l'acide nucléique de la phase solide,
dans lequel, à l'étape (1), le solvant organique hydrosoluble ou la solution contenant le solvant organique hydrosoluble est ajouté(e) séparément en au moins deux lots,
où la quantité de la première addition est comprise entre 5 et 90 %/vol. d'une quantité d'addition totale, et
dans lequel l'étape (1) consiste en outre à :
après l'ajout du solvant organique hydrosoluble ou de la solution contenant le solvant organique hydrosoluble au moins une fois à la solution d'échantillon, agiter la solution d'échantillon par une opération comprenant au moins un élément parmi une agitation, une inversion et un mouvement de rotation ; et ajouter en outre le solvant organique hydrosoluble ou la solution contenant le solvant organique hydrosoluble au moins une fois à la solution d'échantillon après l'agitation.

2. Procédé pour séparer et purifier un acide nucléique selon la revendication 1,
dans lequel l'étape (1) consiste à :
après l'ajout du solvant organique hydrosoluble ou de la solution contenant le solvant organique hydrosoluble au moins une fois à la solution d'échantillon, agiter la solution d'échantillon par une opération comprenant au moins une aspiration et un soutirage de la solution, et
ajouter en outre le solvant organique hydrosoluble ou la solution contenant le solvant organique hydrosoluble au moins une fois à la solution d'échantillon après l'agitation,
moyennant quoi dans le cas où l'on réalise à la fois l'opération comprenant au moins un élément parmi une agitation, une inversion et un mouvement de rotation et l'opération comprenant au moins une aspiration et un soutirage de la solution, ces opérations peuvent être réalisées dans un ordre arbitraire.

3. Procédé pour séparer et purifier un acide nucléique selon l'une quelconque des revendications 1 ou 2,
dans lequel, à l'étape (1), la solution d'échantillon contenant le solvant organique hydrosoluble présente une concentration du solvant organique hydrosoluble comprise dans une plage de 5 à 90 % en masse.

4. Procédé pour séparer et purifier un acide nucléique selon l'une quelconque des revendications 1 ou 2,
dans lequel, à l'étape (1), la solution d'échantillon contenant le solvant organique hydrosoluble présente une concentration du solvant organique hydrosoluble comprise dans une plage de 10 à 60 % en masse.

5. Procédé pour séparer et purifier un acide nucléique selon l'une quelconque des revendications 1 ou 2,
dans lequel, à l'étape (1), la solution d'échantillon contenant le solvant organique hydrosoluble présente une concentration du solvant organique hydrosoluble comprise dans une plage de 20 à 40 % en masse.

6. Procédé pour séparer et purifier un acide nucléique selon l'une quelconque des revendications 1 à 5,
dans lequel la phase solide est une membrane poreuse comprenant un polymère organique qui adsorbe un acide nucléique par une interaction n'impliquant essentiellement aucune liaison ionique.

7. Procédé pour séparer et purifier un acide nucléique selon la revendication 6,
dans lequel le polymère organique possède un groupe hydroxyle.

8. Procédé pour séparer et purifier un acide nucléique selon la revendication 6 ou 7,
dans lequel la membrane poreuse comprend un matériau organique obtenu par la saponification d'un mélange d'acétylcelluloses ayant différentes valeurs d'acétyle.

9. Procédé pour séparer et purifier un acide nucléique selon l'une quelconque des revendications 6 à 8,
dans lequel la membrane poreuse comporte une surface avant et une surface arrière qui sont asymétriques l'une par rapport à l'autre.

10. Procédé pour séparer et purifier un acide nucléique selon l'une quelconque des revendications 1 à 9,
dans lequel la solution de lyse est un réactif de solubilisation d'acide nucléique.

11. Procédé pour séparer et purifier un acide nucléique selon l'une quelconque des revendications 1 à 10,
dans lequel le biomatériau est un tissu animal.

12. Procédé pour séparer et purifier un acide nucléique selon la revendication 10,
dans lequel le réactif de solubilisation d'acide nucléique comprend au moins un élément sélectionné dans le groupe consistant en un sel chaotropique, un agent de stabilisation d'acide nucléique, un tensioactif, un tampon et un agent anti-mousse.

13. Procédé pour séparer et purifier un acide nucléique selon la revendication 12,
dans lequel le sel chaotropique comprend au moins un élément sélectionné dans le groupe consistant en le chlorhydrate de guanidine et le thiocyanate de guanidine.

14. Procédé pour séparer et purifier un acide nucléique selon l'une quelconque des revendications 1 à 13,
dans lequel le solvant organique hydrosoluble est au moins un élément sélectionné dans le groupe consistant en le méthanol, l'éthanol, le propanol ou un isomère de celui-ci et le butanol ou un isomère de celui-ci.

15. Procédé pour séparer et purifier un acide nucléique selon l'une quelconque des revendications 1 à 14,
dans lequel la solution de lavage comprend au moins un élément sélectionné dans le groupe consistant en le méthanol, l'éthanol, le propanol ou un isomère de celui-ci et le butanol ou un isomère de celui-ci, en une quantité comprise entre 20 et 50 % en masse.

16. Procédé pour séparer et purifier un acide nucléique selon l'une quelconque des revendications 1 à 15,
dans lequel la solution de lavage est une solution comprenant un sel de chlorure en une quantité comprise entre 10 mmol/l et 1 mol/l.

17. Procédé pour séparer et purifier un acide nucléique selon l'une quelconque des revendications 8 à 16,
dans lequel, aux étapes (2), (3) et (4), le passage de la solution d'échantillon contenant le solvant organique hydrosoluble, de la solution de lavage ou de la solution de récupération à travers la membrane poreuse est réalisé en utilisant : une cartouche de séparation-purification d'acide nucléique qui reçoit la membrane poreuse, à travers laquelle peut passer une solution, à l'intérieur d'un contenant comportant au moins deux ouvertures ; et un appareil générant une pression qui est une pompe amovible qui peut être montée sur l'une des au moins deux ouvertures de la cartouche de séparation-purification d'acide nucléique.
